# FASCICULE DE BREVET EUROPEEN

(11) **EP 2 652 137 B1**
(45) Date de publication et mention de la délivrance du brevet: **27.09.2017**
(21) Numéro de dépôt: 11807784.1
(22) Date de dépôt: 15.12.2011
(51) Int. Cl.: C12N 15/115, C12Q 1/68, A61K 31/713, A61P 3/00, A61P 9/00

(54) **LIGAND SPECIFIQUE DE LA PROTEINE LAR**
LAR-PROTEINSPEZIFISCHE LIGANDEN
LAR PROTEIN-SPECIFIC LIGAND

(30) Priorité: 16.12.2010 FR 1060619
(43) Date de publication de la demande: 23.10.2013
(73) Titulaire: Commissariat à l'Énergie Atomique et aux Énergies Alternatives, 75015 Paris (FR)
(72) Inventeur: DUCONGE, Frédéric, F-92330 Sceaux (FR); CIBIEL, Agnès, F-91440 Bures sur Yvette (FR)
(74) Mandataire: Cabinet Plasseraud
(86) Numéro de dépôt international: PCT/IB2011/055710
(87) Numéro de publication internationale: WO 2012/080976

(56) Documents cités:
- WO-A1-2005/093097
- WO-A2-2007/041317
- WO-A2-2009/021684
- PESTOURIE ET AL: "Aptamers against extracellular targets for in vivo applications", BIOCHIMIE, vol. 87, no. 9-10, 1 septembre 2005 (2005-09-01), pages 921-930, XP005074124, ISSN: 0300-9084
- TAVITIAN BERTRAND ET AL: "In Vivo Imaging of Oligonucleotidic Aptamers", METHODS IN MOLECULAR BIOLOGY, vol. 535, 1 janvier 2009 (2009-01-01), pages 241-259, XP008138323, ISSN: 1064-3745
- FREISS G ET AL: "Protein tyrosine phosphatases and breast cancer", CRITICAL REVIEWS IN ONCOLOGY / HEMATOLOGY, vol. 52, no. 1, 1 octobre 2004 (2004-10-01), pages 9-17, XP004560866, ISSN: 1040-8428 cité dans la demande
- DEN HERTOG JEROEN ET AL: "Receptor protein-tyrosine phosphatase signalling in development", INTERNATIONAL JOURNAL OF DEVELOPMENTAL BIOLOGY, vol. 43, no. 7, 1999, pages 723-733, XP002652823, ISSN: 0214-6282

## Description

### Domaine de l'invention

La présente invention concerne un ligand spécifique de la protéine LAR, ainsi que son utilisation diagnostique et thérapeutique, ou son utilisation pour la détection *in vitro* de la protéine LAR dans un échantillon biologique.

### Arrière-plan technique

La protéine tyrosine phosphatase apparentée à l'antigène leucocytaire commun, PTP LAR (*Protein Tyrosine Phospatase Leukocyte common Antigen-Related*), ou protéine LAR, connue également sous le nom de protéine tyrosine phosphatase, de type récepteur, F, PTPRF (*Protein Tyrosine Phosphatase, Receptor type, F*), joue un rôle important dans le développement des glandes mammaires (Schaapveld et al. (1997) Dev. Biol. 188:134-146) et dans la tumorigenèse du cancer du sein (Freiss et al. (2004) Critical Reviews in Oncology/Hematology. 52:9-17). Il a été démontré, entre autres, que l'expression de la protéine LAR est augmentée dans les cellules de cancer du sein, notamment transformées avec l'oncogène ErbB-2 (Yang et al. (1999) Mol. Carcinog. 25:139-149 ; LeVea et al. (2000) Breast Cancer Research and Treatment 64:221-228). D'autre part, il semblerait que la protéine LAR soit importante pour la régulation de l'activité de plusieurs récepteurs tyrosine kinase (Kulas et al. (1996) J Biol Chem 271:748-754). La protéine LAR peut également servir de biomarqueur pour prédire la réponse à des traitements anti-EGFR pour le cancer du sein (WO 2009021684).

En outre, cette protéine est un inhibiteur connu des voie d'activation de l'insuline et un niveau d'expression accru de cette protéine a été trouvé dans les tissus insulino-sensibles des obèses et des personnes insulino-résistantes (Ahmad et al. (1995) J Clin Invest 95:2806-2812). Ainsi, il a été démontré que la protéine LAR pouvait contribuer à des pathologies liées à la résistance à l'insuline comme les maladies coronariennes chez les patients atteints de diabète de type 2 (Menzaghi et al. (2008) J Intern Med 263:653-654).

Les ligands de la protéine LAR identifiés jusqu'à ce jour, essentiellement de type immunoglobuline, présentent des limitations, notamment en terme de coût de synthèse, d'immunogénicité ou d'affinité pour leur cible WO2007/041317 propose (notamment p.10) d'utiliser des aptamères contre la protéine LAR, mais n'en divulgue pas d'exemple.

### Résumé de l'invention

La présente invention découle de la mise en évidence inattendue, par les inventeurs, d'un aptamère, de nature nucléotidique, reconnaissant spécifiquement la protéine LAR à la surface de cellules, avec une constante de dissociation de l'ordre du nanomolaire.

Ainsi, la présente invention concerne un aptamère comprenant un acide nucléique capable de reconnaître spécifiquement la protéine LAR à la surface d'une cellule.

Plus particulièrement, l'invention concerne un aptamère comprenant, ou consistant en, un acide nucléique comprenant, ou constitué de :
- la séquence ACUGUCCCAGUAUGACGCGACUGCUUAGGUGGGAUGUUUCCC AUGCCUCG (SEQ ID NO : 1), ou
- une séquence comprenant, ou constituée d'au moins 15 nucléotides consécutifs d'un polynucléotide ayant au moins 60% d'identité avec SEQ ID NO : 1, sous réserve qu'un acide nucléique constitué de cette séquence se lie à la protéine LAR.

Dans un mode de réalisation particulier, l'invention concerne l'aptamère selon l'invention, pour son utilisation à titre de médicament ou d'agent diagnostique.

La présente invention concerne également une composition pharmaceutique ou diagnostique comprenant, à titre de substance active, au moins un aptamère selon l'invention, en association avec au moins un véhicule pharmaceutiquement acceptable.

A ce titre, l'invention concerne également une méthode *in vitro* de diagnostic d'un cancer chez un individu, comprenant les étapes suivantes :
- mettre en contact l'échantillon biologique avec un aptamère selon l'invention ;
- déterminer la quantité d'aptamère fixé dans l'échantillon ;
- éventuellement comparer la quantité d'aptamère fixé dans l'échantillon à au moins une valeur prédéterminée
- en déduire si l'individu est atteint d'un cancer.

La présente invention concerne également une méthode de diagnostic d'un cancer chez un individu, comprenant les étapes suivantes :
- administrer un aptamère selon l'invention à l'individu ;
- détecter, quantifier et/ou localiser l'aptamère dans l'individu ou une partie de l'individu ;
- en déduire si l'individu est atteint d'un cancer.

La présente invention concerne également l'utilisation *in vitro* d'un aptamère selon l'invention, pour détecter la présence de protéine LAR, ou déterminer la quantité de protéine LAR, présente dans un échantillon biologique.

La présente invention concerne une méthode *in vitro* de détection ou détermination de la quantité de protéine LAR dans un échantillon biologique, comprenant les étapes suivantes :
- mettre en contact l'échantillon biologique avec un aptamère selon l'invention ;
- quantifier, ou détecter la présence ou l'absence d'aptamère fixé dans l'échantillon ;
- en déduire la quantité, ou la présence ou l'absence de protéine LAR dans l'échantillon.

La présente invention concerne également une méthode de détection, de quantification ou de localisation de protéine LAR, dans un individu ou une partie d'un individu, comprenant les étapes suivantes :
- administrer un aptamère ou une composition diagnostique tel que défini ci-dessus à l'individu ;
- détecter, quantifier et/ou localiser l'aptamère dans l'individu ou une partie de l'individu ;
- en déduire la présence ou l'absence, la quantité, et/ou la localisation de protéine LAR dans l'individu ou la partie de l'individu.

La présente invention concerne également l'utilisation d'un aptamère tel que défini ci-dessus, pour le criblage de ligands de la protéine LAR.

La présente invention concerne également une méthode de criblage de ligands de la protéine LAR comprenant les étapes suivantes :
- la mise en présence de protéine LAR et, concomitamment ou successivement, d'un ligand à cribler et d'un aptamère tel que défini ci-dessus ;
- déterminer la quantité d'aptamère fixé à la protéine LAR ;
- en déduire si le ligand est un ligand de la protéine LAR.

### Description détaillée de l'invention

Comme on l'entend ici, un « aptamère » désigne un composé, comprenant au moins un acide nucléique, susceptible de se lier de manière spécifique à une cible, notamment de nature protéique, par l'intermédiaire de l'acide nucléique. Un aptamère est dit se lier de manière spécifique à une cible lorsqu'il ne présente essentiellement pas d'affinité pour un composé sans rapport structural avec la cible. De préférence, dans le cas d'une cible protéique, un composé protéique est dit sans rapport structural avec la cible selon l'invention, lorsque l'identité de séquence entre la cible et le composé est inférieure à 60%, de préférence inférieure à 70%, de préférence encore inférieure à 80%. De préférence, selon l'invention, un aptamère est dit ne présenter essentiellement pas d'affinité pour un composé selon l'invention, notamment lorsque la constante de dissociation de l'aptamère vis-à-vis du composé est supérieure à 10⁻⁶ mol/l, de préférence supérieure à 10⁻⁷ mol/l. La constante de dissociation peut notamment être déterminée, dans des conditions standards, à l'aide des représentations de Scatchard et de Lineweaver Burk bien connues de l'homme du métier.

De manière avantageuse, l'aptamère selon l'invention est spécifique de la protéine LAR, en particulier de la protéine LAR humaine, notamment lorsque la protéine LAR est exprimée à la surface d'une cellule. La protéine LAR, ou protéine tyrosine phosphatase apparentée à l'antigène leucocytaire commun, PTP LAR (*Protein Tyrosine Phospatase Leukocyte common Antigen-Related*), connue également sous le nom de protéine tyrosine phosphatase, de type récepteur, F, PTPRF (*Protein Tyrosine Phosphatase, Receptor type, F*) est bien connue de l'homme du métier. La protéine LAR humaine est notamment décrite sous la référence P10586 dans la base de données *GenBank.* A titre d'exemple, la protéine LAR humaine est représentée par la séquence SEQ ID NO : 3.

L'aptamère selon l'invention peut également comprendre au moins un groupement additionnel en plus de l'acide nucléique. Ainsi, l'acide nucléique selon l'invention peut être lié à au moins un groupement additionnel. Par ailleurs, de manière préférentielle, l'aptamère selon l'invention est constitué de l'acide nucléique selon l'invention et d'au moins un groupement additionnel selon l'invention.

Le groupement additionnel selon l'invention peut être de tout type et de toute nature. Le groupement additionnel selon l'invention peut ainsi être un radioisotope, une molécule organique comprenant 100 atomes de carbone au plus, une nanoparticule, notamment une micelle, une protéine, notamment une glycoprotéine, un glucide, un lipide, ou encore un polynucléotide. Selon l'invention, on préfère toutefois que le groupement additionnel selon l'invention soit sélectionné dans le groupe constitué d'un marqueur détectable, d'un composé pharmacologique, et d'un composé susceptible de modifier les caractéristiques pharmacocinétiques d'un acide nucléique auquel il est lié, tel que le polyéthylène glycol (PEG).

Le marqueur détectable selon l'invention peut être de tout type, il peut notamment s'agir d'un fluorophore, par exemple la fluorescéine ou la luciférase ; d'un radioisotope, notamment adapté à la scintigraphie, par exemple ^{99m}Tc ; d'une étiquette reconnaissable par un anticorps, par exemple la protéine c-Myc; d'une étiquette d'affinité, par exemple la biotine ; d'une enzyme, par exemple la peroxydase de raifort.

Le composé pharmacologique selon l'invention peut également être de tout type, il peut notamment s'agir d'un agent de chimiothérapie anticancéreuse, tel qu'un agent cytostatique ou cytolytique. Le composé pharmacologique selon l'invention peut également être de toute nature, il peut notamment s'agir d'un dérivé du platine, d'une molécule organique comprenant moins de 100 atomes de carbone, d'un peptide, d'un analogue de nucléotide, d'une toxine, d'un ARN interférent, ou d'un oligonucléotide antisens.

De préférence, l'acide nucléique selon l'invention est de l'ARN. Comme cela apparaîtra de manière claire à l'homme du métier, il est tout particulièrement préféré que l'acide nucléique selon l'invention soit monobrin. Il est également tout particulièrement préféré que l'acide nucléique selon l'invention ait une structure tridimensionnelle lui permettant de se lier spécifiquement à la protéine LAR. Par ailleurs, le squelette ou le ribose de l'acide nucléique selon l'invention peut être modifié, en totalité ou en partie, notamment pour le rendre résistant à une dégradation hydrolytique, en particulier du fait de l'action de nucléase, notamment lorsque l'acide nucléique est de l'ARN. De telles modifications sont bien connues de l'homme du métier et recouvrent notamment les modifications de la fonction OH sur le carbone en position 2' du ribose par méthylation, ou la substitution de cette fonction OH par un groupement amino ou par un halogène, notamment par le fluor, ainsi que le recours à un squelette phosphorothioate, ou à des structure de type acide nucléique bloqué (*Locked Nucleic Acid,* LNA) ou acide nucléique peptidique (*Peptide Nucleic Acid,* PNA). Ainsi, de manière préférée, l'acide nucléique selon l'invention est un ARN dont les riboses des nucléotides pyrimidines portent un atome de fluor sur le carbone en position 2', les riboses des nucléotides puriques pouvant être inchangés.

Une séquence ayant au moins 60% d'identité en nucléotides avec SEQ ID NO : 1 selon l'invention, diffère notamment de SEQ ID NO : 1 par l'insertion, la suppression ou la substitution d'au moins un nucléotide. Comme on l'entend ici, le pourcentage d'identité entre deux séquences est défini comme le nombre de positions pour lesquelles les bases sont identiques lorsque les séquences sont alignées de manière optimale, divisé par le nombre total de bases de la plus grande des deux séquences. Deux séquences sont dites alignées de manière optimale lorsque le pourcentage d'identité est maximal. Par ailleurs, comme cela apparaitra de manière claire à l'homme du métier, il peut être nécessaire de faire appel à des ajouts de positions lacunaires (des « *gaps* ») de manière à obtenir un alignement optimal entre les deux séquences.

Un acide nucléique est dit se lier à la protéine LAR, si la constante de dissociation de l'acide nucléique vis-à-vis de la protéine LAR, notamment humaine, de préférence exprimée par une cellule, en particulier une cellule de lignée cellulaire MCF-7, A-431, MDA-MB-231, HEK293, HEK293-KDR, 4T1, ou EMT6, comme cela est illustré dans les exemples, est inférieure à 10⁻⁶ mol/l, de préférence inférieure à 10⁻⁷ mol/l, de préférence encore inférieure à 10⁻⁸ mol/l.

Comme cela apparaîtra clairement à l'homme du métier, lorsque l'aptamère selon l'invention comprend un acide nucléique selon l'invention, il peut également comprendre d'autres acides nucléiques. En revanche, lorsque l'aptamère selon l'invention est constitué de l'acide nucléique selon l'invention, il ne comprend pas d'autres acides nucléiques. De manière similaire, lorsque l'acide nucléique selon l'invention comprend une séquence, il peut également comprendre des séquences additionnelles s'étendant du côté 5' et/ou 3' de la séquence en question. En revanche, lorsque l'acide nucléique selon l'invention est constitué d'une séquence, il ne comprend pas de séquences additionnelles en plus de la séquence en question.

Une séquence comprenant SEQ ID NO: 1 selon l'invention peut notamment comprendre des séquences du côté 5' et/ou 3' visant à structurer l'acide nucléique. On préfère ainsi que l'acide nucléique selon l'invention comprenne, ou soit constitué de SEQ ID NO : 2. Dans ce cadre, l'invention concerne alors également, en particulier, un acide nucléique comprenant, ou constitué d'au moins 15 nucléotides consécutifs d'une séquence ayant au moins 60% d'identité avec SEQ ID NO : 2, sous réserve qu'un acide nucléique constitué de cette séquence se lie à la protéine LAR.

De préférence, la séquence comprenant, ou constituée d'au moins 15 nucléotides consécutifs d'une séquence ayant au moins 60% d'identité avec SEQ ID NO : 1 ou 2 selon l'invention, comprend ou est constituée d'au moins 16, 17, 18, 19, 20, 21, 22, 23, 24, 25, 26, 27, 28, 29, 30, 31, 32, 33, 34, 35, 36, 37, 38, 39, 40, 41, 42, 43, 44, ou 45 nucléotides, ou tous les nucléotides, consécutifs d'une séquence ayant au moins 60% d'identité avec SEQ ID NO : 1 ou 2.

De préférence également, la séquence comprenant, ou constituée d'au moins 15 nucléotides consécutifs d'une séquence ayant au moins 60% d'identité avec SEQ ID NO : 1 ou 2 selon l'invention, comprend ou est constituée d'au moins 15 nucléotides consécutif d'une séquence ayant au moins 65%, 70%, 75%, 80%, 85%, 90%, 95%, ou 100%, d'identité avec SEQ ID NO : 1 ou 2 selon l'invention.

De manière plus préférée, la séquence comprenant, ou constituée d'au moins 15 nucléotides consécutifs d'une séquence ayant au moins 60% d'identité avec SEQ ID NO : 1 ou 2 selon l'invention, comprend ou est constituée d'au moins 16, 17, 18, 19, 20, 21, 22, 23, 24, 25, 26, 27, 28, 29, 30, 31, 32, 33, 34, 35, 36, 37, 38, 39, 40, 41, 42, 43, 44, ou 45 nucléotides, ou tous les nucléotides, consécutifs d'une séquence ayant au moins 80% d'identité avec SEQ ID NO : 1 ou 2.

De manière davantage préférée, la séquence comprenant, ou constituée d'au moins 15 nucléotides consécutifs d'une séquence ayant au moins 60% d'identité avec SEQ ID NO : 1 ou 2 selon l'invention, comprend ou est constituée d'au moins 16, 17, 18, 19, 20, 21, 22, 23, 24, 25, 26, 27, 28, 29, 30, 31, 32, 33, 34, 35, 36, 37, 38, 39, 40, 41, 42, 43, 44, ou 45 nucléotides, ou tous les nucléotides, consécutifs d'une séquence ayant au moins 85% d'identité avec SEQ ID NO : 1 ou 2.

De manière encore plus préférée, la séquence comprenant, ou constituée d'au moins 15 nucléotides consécutifs d'une séquence ayant au moins 60% d'identité avec SEQ ID NO : 1 ou 2 selon l'invention, comprend ou est constituée d'au moins 16, 17, 18, 19, 20, 21, 22, 23, 24, 25, 26, 27, 28, 29, 30, 31, 32, 33, 34, 35, 36, 37, 38, 39, 40, 41, 42, 43, 44, ou 45 nucléotides, ou tous les nucléotides, consécutifs d'une séquence ayant au moins 90% d'identité avec SEQ ID NO : 1 ou 2.

De manière particulièrement préférée, la séquence comprenant, ou constituée d'au moins 15 nucléotides consécutifs d'une séquence ayant au moins 60% d'identité avec SEQ ID NO : 1 ou 2 selon l'invention, comprend ou est constituée d'au moins 16, 17, 18, 19, 20, 21, 22, 23, 24, 25, 26, 27, 28, 29, 30, 31, 32, 33, 34, 35, 36, 37, 38, 39, 40, 41, 42, 43, 44, ou 45 nucléotides, ou tous les nucléotides, consécutifs d'une séquence ayant au moins 95% d'identité avec SEQ ID NO : 1 ou 2.

Par ailleurs, de manière alternative, la séquence comprenant, ou constituée d'au moins 15 nucléotides consécutifs d'une séquence ayant au moins 60% d'identité avec SEQ ID NO : 1 ou 2 selon l'invention, peut être constituée d'une séquence de 95 nucléotides présentant au moins 60%, 65%, 70%, 75%, 80%, 85%, 90% ou 95% d'identité avec SEQ ID NO : 2 et un acide nucléique constitué de cette séquence est susceptible d'adopter la structure de formule (I) suivante : dans laquelle :
- A, C, G, U sont les ribonucléotides Adenosine, Cytidine, Guanosine, et Uridine ;
- chacun des X et des Y, identiques ou différents, représente A, C, G ou U ;
- chacune des paires X-Y ou Y-X, identiques ou différentes, représentent des paires A-U, U-A, G-C, C-G, G-U ou U-G.

L'homme du métier peut aisément déterminer la structure secondaire susceptible d'être adoptée par un acide nucléique ayant une séquence selon l'invention, par exemple à l'aide d'algorithmes ou de logiciels de modélisation bien connus de l'homme du métier, tels que *mfold* (version 3.4) décrit dans Nucleic Acids Res. (2003) 31:3406-15 et *vsfold5 RNA Pseudoknot Prediction* décrit dans One (2007), 2:905. A ce titre, les inventeurs ont établi, à l'aide de tels outils de modélisation, que l'aptamère V8 de séquence SEQ ID NO : 2, qui est décrit dans les Exemples, adopte une structure de formule (I).

Lorsque l'aptamère selon l'invention est utilisé à titre de médicament ou est compris dans une composition pharmaceutique, il est notamment utile pour inhiber l'angiogenèse, ainsi que pour la prévention ou le traitement, notamment en inhibant l'angiogenèse, des cancers, et des maladies impliquant une néovascularisation oculaire, telles que la dégénérescence maculaire liée à l'âge et la rétinopathie diabétique. Par ailleurs, lorsqu'il est utilisé à titre de médicament ou est compris dans une composition pharmaceutique, l'aptamère selon l'invention est également utile pour le traitement des maladies métaboliques, en particulier de l'insulino-résistance, du diabète, notamment du diabète de type 2, du syndrome métabolique ou des maladies cardiovasculaires, telles que les maladies coronariennes, notamment chez les patients atteints de diabète de type 2.

Lorsque l'aptamère selon l'invention est utilisé à titre d'agent diagnostique ou est compris dans une composition diagnostique, il est notamment utile pour le diagnostic des cancers ou des maladies métaboliques, telles que l'insulino-résistance.

De préférence, dans les méthodes de détection ou de diagnostic selon l'invention la détection d'aptamère fixé dans l'échantillon est effectuée par mise en oeuvre d'une réaction de polymérisation en chaîne (PCR) destinée à amplifier l'aptamère, notamment une RT-PCR (PCR comprenant une étape de transcription inverse). De préférence, lorsque l'acide nucléique selon l'invention comprend SEQ ID NO : 2, la PCR est mise en oeuvre à l'aide du couple d'amorces de séquences CTTGTCATCAACCTGCCAGCCAGT (SEQ ID NO : 4) et GGGAGATGCTCTGTCAGACTACG (SEQ ID NO : 5) ou du couple d'amorce ayant les séquences complémentaires de SEQ ID NO : 4 et SEQ ID NO : 5. Par ailleurs, l'invention concerne également un acide nucléique comprenant ou constitué de SEQ ID NO : 4 ou de SEQ ID NO : 5 ou des séquences complémentaires de SEQ ID NO : 4 ou SEQ ID NO : 5.

De préférence également, dans les méthodes de détection ou de diagnostic selon l'invention, lorsque l'aptamère est administré à un individu, on préfère que l'aptamère soit détectable par des méthodes d'imagerie *in vivo,* notamment externes, telles que l'imagerie fluorescente planaire ou tridimensionnelle (3D), ou internes, telles que l'endoscopie par exemple.

Par ailleurs, la présente invention concerne également un ligand de la protéine LAR pour son utilisation comme inhibiteur de l'angiogenèse, ainsi que pour la prévention ou le traitement, notamment en inhibant l'angiogenèse, des cancers, et des maladies impliquant une néovascularisation oculaire, telles que la dégénérescence maculaire liée à l'âge et la rétinopathie diabétique. Le ligand de la protéine LAR selon l'invention peut être de tout type, il peut notamment s'agir d'un anticorps ou d'un fragment d'anticorps comprenant la partie de liaison à l'antigène ou paratope, un fragment Fv mono-chaîne (scFv), ou encore un aptamère, notamment, comme cela a été indiqué ci-dessus, un aptamère selon l'invention.

### Description des figures

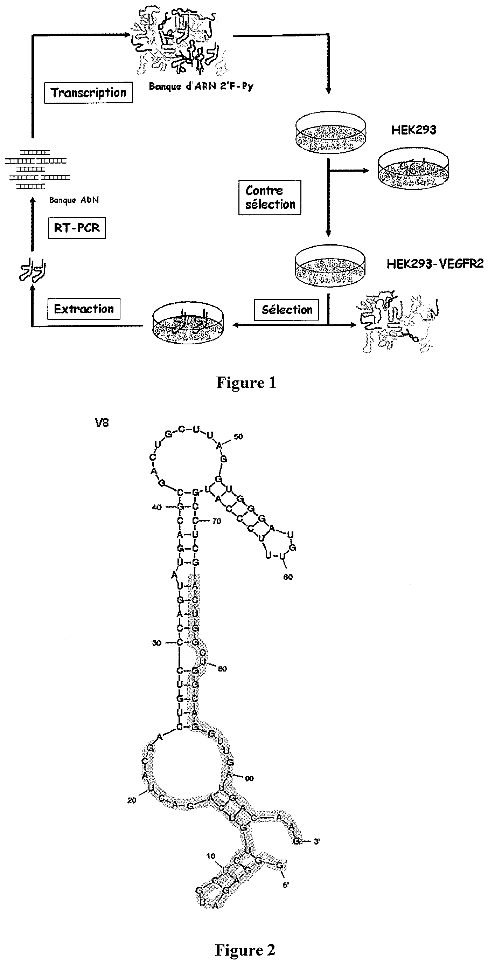

Figure 1 : schéma du protocole de sélection
   La figure 1 représente le protocole de sélection des aptamères utilisé par les inventeurs pour obtenir l'aptamère V8.
Figure 2 : Prédiction de structure de l'aptamère V8
   La figure 2 représente la structure secondaire de l'aptamère V8 prédite à l'aide du programme mfold (version 3.4, http://mfold.bioinfo.rpi.edu/cgi-bin/rna-form1.cgi). Sur cette structure, les séquences fixes permettant une amplification par PCR ont été surlignées en gris.

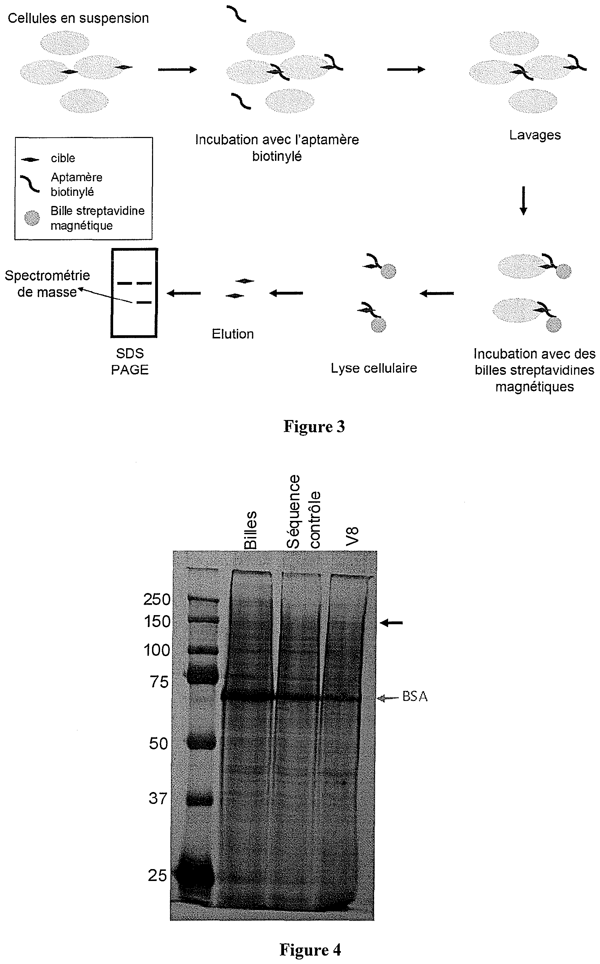

Figure 3 : Protocole d'identification de la cible de l'aptamère V8
   La figure 3 représente le protocole d'identification de la cible de l'aptamère V8. Brièvement, l'aptamère biotinylé est incubé sur les cellules MCF-7 en suspension. Après plusieurs lavages, des billes magnétiques couplées à la streptavidine sont ajoutées. Seules les cellules qui ont fixé l'aptamère V8 biotinylé vont être retenues sur les billes. Les cellules sont alors lysées et les protéines retenues par l'aptamère V8 sont éluées. Les protéines sont alors déposées sur gel SDS-PAGE et les bandes spécifiques sont analysées par spectrométrie de masse.
Figure 4 : Migration des protéines par électrophorèse SDS-PAGE
   La figure 4 est une photographie d'un gel SDS-PAGE sur lequel sont mis à migrer les éluats provenant de billes mises directement en contact avec les cellules (billes), d'un oligonucléotide contrôle (séquence contrôle), ou de l'aptamère V8. La principale bande correspondant à l'éluat provenant de l'aptamère est indiquée. La bande correspondant à l'Albumine Sérique Bovine (BSA), utilisée comme compétiteur non spécifique, est également représentée. Brièvement, les protéines sont déposées sur un gel SDS PAGE 10%. Le gel est ensuite coloré à l'aide du kit protéosilver silver stain kit (sigma). Les bandes intéressantes sont découpées, décolorées avec le même kit et analysées en spectrométrie de masse.

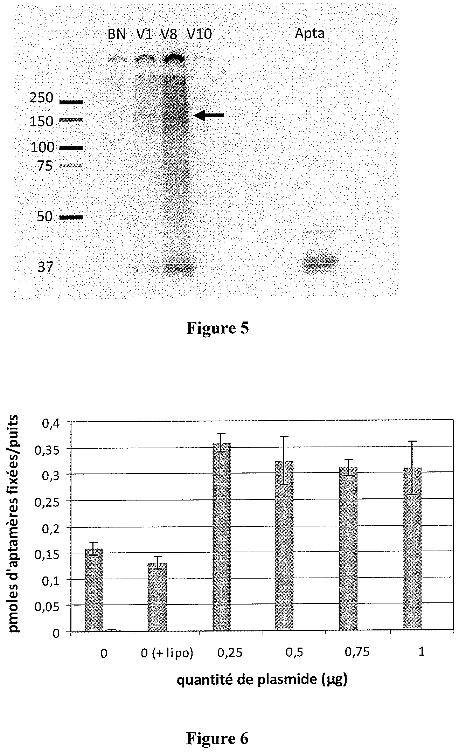

Figure 5 : Pontage aux UV des aptamères V1, V8 et V10
   La figure 5 est une autoradiographie d'un gel SDS-PAGE à 10% sur lequel sont mis à migrer des lysats cellulaires de cellules HEK293-KDR mise en présence des aptamères V1, V8 et V10 ou la banque naïve (BN) radiomarqués et pontés par irradiation UV. L'aptamère seul, quant à lui, migre vers 37 kDa (piste Apta). La flèche indique la principale protéine pontée par l'aptamère V8, dont la taille correspond à celle de la protéine LAR. Brièvement, les aptamères et la banque naïve ont été incubés sur les cellules à une concentration de 25 nM. Après 15 minutes d'incubation à 37°C, les séquences non associées aux cellules ont été éliminées. Le pontage aux UV a été réalisé avec une énergie d'irradiation de 800 000 µJ/cm², puis les cellules ont été lysées et les protéines ont été déposées sur un gel 10%.
Figure 6 : Effet de la surexpression de la protéine LAR sur la fixation de l'aptamère V8
   La figure 6 représente la quantité d'aptamères V8 fixés par puits (axe des ordonnées, pmoles) contenant des cellules HEK293 non transfectées (0), non transfectées et mise en présence de lipofectamine (0 (+ lipo)), ou transfectées par 0,25 µg, 0,5 µg, 0,75 µg ou 1 µg de plasmide exprimant la protéine LAR (axe des abscisses). L'aptamère radiomarqué est incubé à une concentration de 10 nM pendant 15 minutes. Après plusieurs lavages, la radioactivité fixée aux cellules est comptée et la quantité d'aptamères fixés est déterminée (en pmoles/cupule).

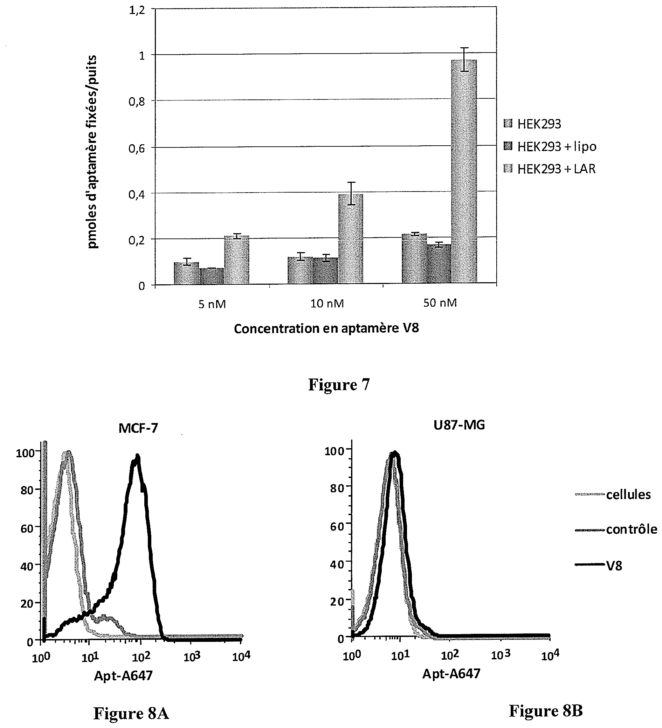

Figure 7 : Effet de la surexpression de la protéine LAR sur la fixation de l'aptamère V8
   La figure 7 représente la quantité d'aptamères V8 fixés par puits (axe des ordonnées, pmoles) contenant des cellules HEK293 non transfectées (0), non transfectées et mise en présence de lipofectamine (0 (+ lipo)), ou transfectées par un plasmide exprimant la protéine LAR, en présence de 5 nM, 10 nM et 50 nM d'aptamère V8 (axe des abscisses). Les aptamères radiomarqués sont incubés pendant 15 minutes. Après plusieurs lavages, la radioactivité fixée aux cellules est comptée et la quantité d'aptamères fixés est déterminée (en pmoles/cupule).
Figures 8A et 8B : Etude de la fixation de l'aptamère V8 sur les cellules MCF-7 et U87-MG en cytométrie de flux
   Les figures 8A et 8B représentent la quantité de cellules MCF-7 (figure 8A) et U-87MG (figure 8B) triées en cytométrie de flux (axe des ordonnées, en pourcentage de la fluorescence maximale) en fonction de la fluorescence émise par l'aptamère V8 (courbe noire), la séquence contrôle (courbe gris foncée), et les cellules seules (courbe gris claire) (axe des abscisses, unités arbitraires). Les cellules ont été incubées avec les aptamères à une concentration de 5 nM pendant 30 minutes, lavées et analysées en FACS. 100 000 événements ont été comptés pour chaque condition.

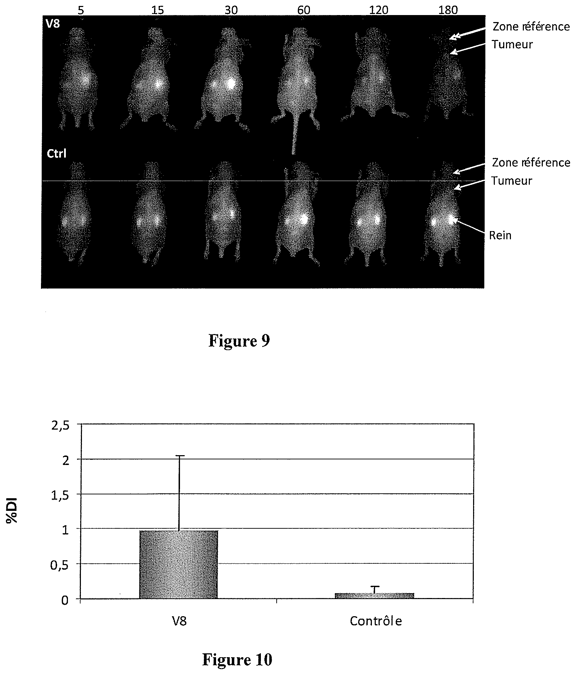

Figure 9: Biodistribution de aptamère V8 et d'une séquence contrôle dans des souris « nude » ayant développé des tumeurs après une xénogreffe de cellules MCF-7
   La figure 9 représente des images acquises au Tomofluo 3D de souris « *nude* » ayant développé des tumeurs après une xénogreffe de cellules MCF-7 auxquelles ont été injectées par voie intraveineuse 1,4 nmoles d'aptamère V8 (partie supérieure de la figure, V8) ou de séquence contrôle (partie inférieure de la figure, Ctrl) marqués à l'Alexa Fluor 680. Les images ont été acquises entre 5 et 180 minutes après injection. La tumeur ainsi que la zone de référence qui permet de mesurer le rapport signal dans la tumeur/référence interne sont indiquées.
Figure 10: Pourcentage de la dose injectée d'aptamère V8 et d'une séquence contrôle présente dans des tumeurs après xénogreffe de cellules MCF-7
   L'aptamère V8 ou une séquence contrôle marqués avec de l'Alexa Fluor 680 ont été injectés dans des souris développant des tumeurs suite à une injection sous cutanée de cellules MCF-7. Trois heures post-injection, le signal fluorescent dans la tumeur a été mesuré par tomographie fluorescente à l'aide d'un appareil TomoFluo3D. La figure 10 représente la quantité de fluorescence déterminée en triplicat en pourcentage de la dose injecté (%DI, axe des ordonnées) dans la tumeur pour l'aptamère V8 et une séquence contrôle.

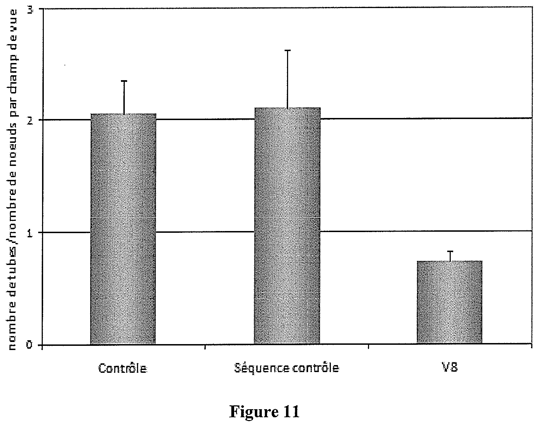

Figure 11 : Effet de l'aptamère V8 sur la formation de tubes endothéliaux par des HUVEC
   Les cellules HUVEC ont été cultivées en présence ou non de 5µM d'aptamère V8 ou de séquence contrôle. Après 16 heures d'incubation à 37°C et 5% de CO₂, la formation des tubes a été évaluée par microscopie en contraste de phase. Le rapport entre le nombre de tubes et le nombre de noeuds visualisés par champ de vue a été calculé. La figure 11 représente le nombre de tubes par noeud de cellules sur quatre champs de vue choisis aléatoirement (axe des ordonnées), en l'absence d'oligonucléotide (contrôle), en présence d'une séquence contrôle, et en présence de l'aptamère V8 (axe des abscisses).

### EXEMPLES

### Exemple 1 : obtention de l'aptamère V8 spécifique de la protéine LAR

### 1- Identification de l'aptamère V8 après 17 tours de sélection sur les cellules HEK293-VEGFR2

Les inventeurs ont utilisé la technique de SELEX, notamment décrite dans la demande WO 2005/093097, dans le but de sélectionner des aptamères contre le récepteur VEGFR2 (vascular endothelial growth factor receptor 2). Pour cela, des cellules HEK293 surexprimant VEGFR2 et pour la contre-sélection des cellules HEK293 n'exprimant pas VEGFR2 (**Figure 1**).

Au cours de ce SELEX, les inventeurs ont fait varier différents paramètres dans le but d'augmenter progressivement la pression de sélection (**Tableau 1**). Par rapport au protocole décrit dans la demande WO 2005/093097, les inventeurs ont apporté quelques modifications : durant quelques tours, les inventeurs ont changé de type cellulaire dans le but d'augmenter la spécificité. Pour ces tours, les inventeurs ont utilisés des cellules endothéliales aortiques de porc (PAE) transfectées de manière stable pour exprimer le VEGFR2 (PAE-VEGFR2) pour la sélection, et non transfectées (PAE) pour la contre-sélection (tours 13 et 14). De plus, les inventeurs ont inversé l'étape de sélection et de contre-sélection (tours 16 et 17). Enfin, tout au long du processus de sélection, du VEGF, le ligand du récepteur VEGFR2, a été ajouté 10 minutes avant la fin de l'incubation avec la banque, à chaque tour de SELEX. Ceci dans le but de sélectionner des aptamères qui ne soient pas décrochés par le VEGF. Les conditions de sélection sont présentées dans le **Tableau 1** ci-dessous :

**Tableau 1 : évolution de la pression de sélection au cours du SELEX.**

| cycle | Type cellulaire | CS : Nb de cellules (millions) | S : Nb de cellules (millions) | Inversion CS et S | Volume d'incubation (mL) | [ ] en ARN 2'F-Py(µM) | Temps d'Incubation (min) | Node lavages | Conditon particulière de lavages | tRNA |
|---|---|---|---|---|---|---|---|---|---|---|
| 1 | HEK293 | 10 | 10 | | 3 | 1 | 30 | 3 | | |
| 2 | HEK293 | 10 | 10 | | 3 | 1 | 30 | 3 | | |
| 3 | HEK293 | 10+10 | 10 | | 3 | 1 | 25 | 3 | | |
| 4 | HEK293 | 5+5 | 5 | | 3 | 1 | 25 | 4 | | |
| 5 | HEK293 | 5+5 | 5 | | 3 | 1 | 20 | 4 | | |
| 6 | HEK293 | 5+5 | 5 | | 3 | 1 | 20 | 5 | | |
| 7 | HEK293 | 5+5 | 5 | | 3 | 1 | 20 | 5 | | |
| 8 | HEK293 | 5+5 | 5 | | 3 | 1,5 | 20 | 5 | L5:5min | |
| 9 | HEK293 | 5+5 | 5 | | 3 | 1,5 | 20 | 5 | L5:5min | |
| 10 | HEK293 | 5+5 | 5 | | 3 | 1 | 20 | 5 | L5:5min | |
| 11 | HEK293 | 5+5 | 5 | | 3 | 1,5 | 20 | 5 | L5:5min | + |
| 12 | HEK293 | 5+5 | 5 | | 3 | 1 | 20 | 5 | L5:5min | + |
| 13 | PAE | 5+5 | 5 | | 3 | 2 | 20 | 5 | L5:5min | + |
| 14 | PAE | 5+5 | 5 | | 3 | 0,5 | 20 | 5 | L5:5min | + |
| 15 | HEK293 | 5+5 | 5 | | 3 | 2 | 20 | 5 | L5:5min | + |
| 16 | HEK293 | 5 | 5 | + | 3 | 2 | 20 | 5 | L5:5min | + |
| 17 | HEK293 | 5 | 5 | + | 3 | 1,5 | 20 | 5 | L5:5min | + |

| | | | | | | | | | | |
|---|---|---|---|---|---|---|---|---|---|---|
| S : Sélection, CS : Contre-sélection, L : lavage. | | | | | | | | | | |

La banque issue du tour 17 a été clonée et séquencée. Les inventeurs ont testé l'affinité d'une trentaine de séquences pour les cellules HEK293-VEGFR2 à 25 nM. Ceci leur a permis d'identifier 3 aptamères particulièrement intéressants avec une forte affinité pour ces cellules, V1, V8 et V10. Cependant, ces aptamères se fixent également sur les cellules HEK293, qui n'expriment pas la cible, indiquant que la cible de ces aptamères n'est pas VEGFR2. Les inventeurs ont alors choisi de poursuivre la caractérisation de l'aptamère V8 qui présente la plus forte affinité pour les cellules (Kd ∼ 5,3 nM).

### 2. Séquence de l'aptamère V8 :

La séquence de l'aptamère V8 est la suivante :

Il est à noter qu'afin d'améliorer la résistance de l'aptamère V8 aux nucléases, les riboses des pyrimidines portent un atome de fluor sur le carbone en position 2' (les riboses des purines sont quant à eux porteurs, comme c'est le cas dans l'ARN naturel, d'une fonction hydroxyle (OH) sur le carbone en position 2').

La **Figure 2** représente une prédiction de structure secondaire réalisée par le logiciel mfold (version 3.4) (Nucleic Acids Res. (2003) 31:3406-15).

### 3. Validation de l'utilisation de l'aptamère V8 pour établir un profil d'expression à la surface de différentes lignées cellulaires.

L'affinité et le Cmax de l'aptamère V8 ont été déterminés pour différentes lignées de cellules cancéreuses (**Tableau 2**).

**Tableau. 2 : Affinité et nombre de cibles par cellule de l'aptamère V8 pour différentes lignées cellulaires**

| espèce | Type cellulaire | Kd (nM) | Cmax (pM) | Nombre de cibles/cellule |
|---|---|---|---|---|
| Homme | MCF-7 | 1,6 +/- 0,6 | 251 +/- 40 | 138 100 +/- 21800 |
| | A-431 | 5,6 +/- 0,6 | 455 +/- 21 | 134 300 +/- 6 100 |
| | MDA-MB-231 | 3 +/- 0,4 | 131 +/- 12 | 77 800 +/- 7 200 |
| | U87-MG | - | - | |
| | HEK293 | 3,8 +/- 0,9 | 264 +/- 52 | 80 000 +/- 15 600 |
| | HEK293-KDR | 5,3 +/- 1,5 | 119 +/- 6 | 46 000 +/- 2 200 |
| Souris | 4T1 | 2,9 +/- 0,1 | 167 +/- 13 | 65 200 +/- 4 900 |
| | EMT6 | 2,8 +/- 0,6 | 129 +/- 24 | 42 400 +/- 8 000 |
| Rat | PC12-MEN-2A | 34,9 +/- 4,4 | 764 +/-112 | 141 400 +/- 20 800 |

| | | | | |
|---|---|---|---|---|
| Pour chaque type cellulaire, les courbes de binding ont été obtenues. A partir du binding spécifique, et à l'aide de la représentation de Scatchard, le Kd et le Cmax ont été calculés pour chaque type cellulaire. En connaissant le Cmax et le nombre de cellules pendant le binding, les inventeurs ont pu en déduire un nombre approximatif de cibles par cellule | | | | |

L'aptamère V8 a une affinité d'environ 3 nM pour la plupart des lignées cellulaires, excepté pour les cellules PC12-MEN-2A pour lesquelles il a une affinité de 35 nM et les cellules U87-MG pour lesquelles il n'a aucune affinité. De façon intéressante, la cible de l'aptamère V8 semble très abondante à la surface de la lignée de cancer du sein MCF-7 et à la surface de la lignée A-431 de cancer de l'épiderme sur-exprimant EGFR.

### 4. Identification de la cible de l'aptamère V8

### a. protocole de purification de la cible de l'aptamère

Afin d'identifier la cible de l'aptamère V8, un protocole dérivé de Berezovski et al. (2008) J. Am. Chem. Soc.*, Aptamer-Facilitated Biomarker Discovery* (AptaBiD) a été utilisé, dans lequel de la BSA a été rajoutée comme compétiteur pour éviter la fixation non spécifique de protéines sur les billes. Le protocole est résumé dans la **Figure 3**.

### b. Gel SDS-PAGE et spectrométrie de masse

Les protéines éluées à la fin du protocole résumé dans la **Figure 3** ont été analysées par électrophorèse **(****Figure 4****).** Une bande spécifique de l'aptamère V8 a été identifiée. Cette bande a été découpée et analysée par spectrométrie de masse. Les peptides d'une vingtaine de protéines ont été détectés dans le fragment de gel. Parmi elles, une protéine a été identifiée comme étant susceptible de correspondre à la cible de l'aptamère V8. Cette protéine a été identifiée comme étant la " *protein tyrosine phosphatase, receptor-type, F*" (PTPRF) également appelé "*leukocyte common antigen-related*" (LAR). Il s'agit d'une protéine membranaire de 213 kDa.

### c. Validation de la cible de l'aptamère V8

Afin de vérifier que l'aptamère V8 interagit avec la protéine LAR, les inventeurs ont tout d'abord réalisé des expériences de pontage aux UV.

Les irradiations aux UV peuvent induire la formation de liaisons covalentes au niveau des points de contact existants entre une protéine et un acide nucléique (Zhang et al. (2004) Biochem Biophys Res Commun 322:705-711).

Les aptamères V1, V8, V10, ainsi que la banque naïve, radiomarqués ont été incubés pendant 15 minutes sur les cellules puis les cellules ont été irradiées aux UV. Les protéines ont ensuite été extraites et déposées sur un gel SDS PAGE 10%. De façon intéressante, l'aptamère V8 a été ponté à une protéine dont la taille correspond à celle de la protéine LAR (**Figure 5**).

Une deuxième validation a été effectuée en utilisant un plasmide d'expression de l'isoforme la plus longue de la protéine LAR. Des cellules HEK293 ont été utilisées pour réaliser la transfection à l'aide de lipofectamine 2000 (*Invitrogen*). Plusieurs quantités de plasmide ont été transfectées (0,25 µg - 1µg). 48 heures après, 10 nM d'aptamère V8 ou de séquence contrôle radiomarqués ont été incubés avec les cellules. Après plusieurs lavages, la radioactivité associée aux cellules a été comptée.

L'aptamère V8 présente de l'affinité pour les cellules HEK293 (**Tableau 2** et **Figure 6**). Cependant, la fixation de l'aptamère V8 est au minimum deux fois plus importante lorsque ces cellules ont été transfectées par le plasmide d'expression de la protéine LAR. On observe que la fixation n'augmente pas lorsque la quantité de plasmide transfecté est augmentée, ce qui indique que 0,25 µg de plasmide est suffisant pour avoir une expression maximale.

Lorsque les cellules ont été traitées avec de la lipofectamine sans ajout de plasmide, la fixation de l'aptamère V8 est identique à celle obtenue avec des cellules non traitées, ce qui indique que l'augmentation de fixation est bien due à l'expression du plasmide et non à la présence de la lipofectamine.

Afin de confirmer ce résultat, l'expérience a été reproduite, en transfectant 0,25 µg de plasmide et en testant différentes concentrations d'aptamère V8 (**Figure 7**).

Comme précédemment, une augmentation de la fixation de l'aptamère V8 est observée lorsque les cellules ont été transfectées avec le plasmide exprimant la protéine LAR. Cette augmentation de fixation est visible quelle que soit la concentration d'aptamère utilisée. On remarque que plus la concentration d'aptamères incubés est élevée, plus l'aptamère se fixe de façon importante par rapport aux cellules non transfectées (5 fois plus de fixation à 50 nM contre 2 fois plus à 5 nM).

Tous ces résultats démontrent que la cible de l'aptamère V8 doit être la protéine LAR.

### Exemple 2 : utilisation de l'aptamère V8 en FACS

L'aptamère V8 et la séquence contrôle (SEQ ID NO: 6), marqués avec de l'AlexaFluor 647 (*Invitrogen*), ont été incubés avec les cellules MCF-7 et les cellules U87-MG (**Figure 8A et 8B**).

Que cela soit sur les cellules MCF-7 ou U87-MG, on observe que la séquence contrôle ne se fixe pas sur les cellules puisque l'intensité de fluorescence est identique à celle obtenue pour les cellules seules. L'aptamère V8 se fixe sur les cellules MCF-7, mais aucune augmentation de fluorescence n'est observée sur les cellules U87-MG. Ces résultats sont en accord avec les expériences de liaison décrites ci-dessus.

### Exemple 3 : utilisation de l'aptamère V8 en imagerie

Afin d'évaluer si l'aptamère V8 peut permettre un ciblage tumoral *in vivo,* les inventeurs ont réalisé des expériences d'imagerie par fluorescence chez la souris. Pour cela, les inventeurs ont utilisé un appareil d'imagerie (*TomoFluo3D*, LETI, Grenoble, France). Cet appareil permet des mesures semi-quantitatives par imagerie planaire et quantitatives par tomographie (Garofalakis et al. (2010) Optics Letters 35:3024-3026). Ces expériences ont été réalisées avec des souris athymiques "*nude*"*.* Des cellules MCF-7 ont été utilisées afin de réaliser des xénogreffes chez ces souris. De 2 semaines à 2 mois après l'injection des cellules en sous-cutanée au niveau du haut du dos, les xénogreffes obtenues atteignent des volumes de l'ordre de la centaine de mm³. Des expériences de biodistribution des aptamères peuvent alors être réalisées par imagerie optique.

La biodistribution de l'aptamère V8 a été comparée à celle d'une séquence contrôle dans des xénogreffes MCF-7. Pour cela, les séquences marquées à l'Alexa Fluor 680 (1,4 nmoles) ont été injectées aux souris dans la veine caudale. L'imagerie planaire a été réalisée entre 1 minute et 3 heures après l'injection et l'imagerie tridimensionnelle a été réalisée dans les tumeurs au temps 3 heures afin de pouvoir quantifier la quantité d'aptamères parvenue dans la tumeur.

### 1. Imagerie planaire

Suite à l'injection de l'aptamère V8 ou de la séquence contrôle en intraveineuse, des images ont été prises à différents temps post-injection. Pour chaque séquence, 3 souris ont été injectées. La **Figure 9** représente respectivement les biodistributions de l'aptamère V8 et de la séquence contrôle dans une souris.

Comme attendu, les séquences sont rapidement éliminées par les urines et par le système hépatobiliaire. Par ailleurs, à partir de ces images obtenues, et pour chacune des souris, des régions d'intérêt (ROI) ont été tracées au niveau de la tumeur et au niveau de la tête entre les deux oreilles (zone de référence) à l'aide du logiciel *ImageJ* (*MacBiophotonics*)*.* L'intensité moyenne de fluorescence a alors été calculée pour chaque ROI. Le bruit de fond a été soustrait (autofluorescence de la souris mesurée à l'aide d'une photo prise avant injection) et le rapport entre la fluorescence dans la tumeur et la fluorescence dans la tête a été calculé au cours du temps. Les moyennes de ces rapports au cours du temps sont indiquées dans le **Tableau 3.**

**Tableau 3 : Rapport de l'intensité de fluorescence dans la tumeur/zone de référence au cours du temps**

| Temps (min) | Contrôle | V8 | |
|---|---|---|---|
| 5 | 1,10 +/- 0,12 | 1,29 +/- 0,12 | ns |
| 10 | 1,17 +/- 0,10 | 1,39 +/- 0,09 | ns |
| 15 | 1,18 +/- 0,04 | 1,51 +/- 0,04 | * |
| 30 | 1,32 +/- 0,08 | 1,66 +/- 0,07 | * |
| 60 | 1,42 +/- 0,09 | 1,73 +/- 0,09 | ns |
| 90 | 1,43 +/- 0,18 | 1,77 +/- 0,18 | * |
| 120 | 1,52 +/- 0,11 | 2,01 +/- 0,11 | ns |
| 150 | 1,54 +/- 0,15 | 1,80 +/- 0,15 | ns |
| 180 | 1,57 +/- 0,17 | 2,25 +/- 0,17 | ns |

| | | | |
|---|---|---|---|
| Les résultats représentent la moyenne des rapports de fluorescence tumeur/zone de référence obtenus à différents temps pour 3 souris. * correspond à un écart significatif du ratio entre l'aptamère et la séquence contrôle mesuré par un test de Student avec P<0.05. ns : non significatif | | | |

De façon intéressante, le rapport tumeur/zone de référence augmente plus rapidement pour l'aptamère V8 que pour la séquence contrôle et cette différence significative est observée dès 15 minutes après injection (1,51 +/- 0,04 pour l'aptamère V8 et 1,18 +/- 0,04 pour la séquence contrôle). Après 15 minutes, le rapport augmente progressivement de façon similaire pour l'aptamère et la séquence contrôle et la différence entre l'aptamère et la séquence contrôle reste alors constante au cours du temps.

### 2. Imagerie tridimensionnelle

Trois heures après l'injection, la quantité d'aptamères accumulée dans les tumeurs a été quantifiée par tomographie. Après reconstruction, l'intensité de fluorescence est visualisée en trois dimensions dans les tumeurs.

Trois heures après injection, les zones correspondant aux tumeurs ont été scannées à l'aide de l'appareil *Tomofluo3D.* Après reconstruction à l'aide du logiciel *Imo3D,* l'intensité de fluorescence a été visualisée en trois dimensions.

Il apparait ainsi que l'intensité de fluorescence est plus importante dans les tumeurs provenant des souris injectées avec l'aptamère V8, par rapport aux souris injectées avec la séquence contrôle. L'accumulation dans les tumeurs 3 heures après injection est donc plus importante pour l'aptamère V8 suggérant que cette accumulation est spécifique, provenant de la liaison de l'aptamère sur sa cible, la protéine LAR.

A l'aide des courbes de calibration réalisées par une fusion tomographie par émission de positons (PET) / tomographie optique diffuse de fluorescence (fDOT), il est possible de quantifier une sonde marquée à l'Alexa Fluor 680 présente à des concentrations comprises entre 3 nM à 1 µM (Garofalakis et al. (2010) Optics Letters 35:3024-3026). Le pourcentage de la dose injectée (%DI) présente dans les tumeurs trois heures après injection a donc pu être déterminée ainsi **(****Figure 10****).**

Trois heures après injection, 0,97 +/- 1,1 %DI d'aptamère V8 sont présents dans la tumeur contre 0,07 +/- 0,11 %DI pour la séquence contrôle. L'accumulation de l'aptamère est donc environ 10 fois plus importante que celle de l'aptamère contrôle. Il est à noter que la différence observée en imagerie tridimensionnelle est plus importante qu'en imagerie planaire.

### Exemple 4 : utilisation de l'aptamère V8 pour inhiber l'angiogenèse

Les inventeurs ont testé si l'aptamère V8 pouvait perturber le processus d'angiogenèse en utilisant un test de formation de tubes endothéliaux *in vitro.* Aucun rôle de la protéine LAR n'est connu à ce jour dans l'angiogénèse, néanmoins, il a été démontré que la protéine LAR peut être impliquée dans la régulation des contacts cellule-cellule épithéliale, ainsi que dans le contrôle des voies de signalisation de la bêta-caténine.

Des cellules endothéliales humaines de la veine ombilicale (HUVEC) ont été cultivées sur du Matrigel™ dans un milieu à faible teneur en hormone de croissance comportant 2% de sérum de veau foetal et du facteur de croissance de fibroblastes basique recombinant (bFGF à 3 ng/mL) (*Invitrogen*). Dans ces conditions, les HUVEC forment un réseau de tubes endothéliaux qui peut être observé par microscopie et représente un modèle *in vitro* de l'angiogénèse. L'effet de l'aptamère V8 sur la formation de ce réseau a été évalué (**Figure 11**).

On observe qu'après 16 heures d'incubation, un réseau de tubes s'est formé pour la condition contrôle avec une moyenne de deux tubes formés par noeud. L'aptamère V8 inhibe la formation des tubes avec une moyenne de 0,72 tubes par noeud alors que la séquence contrôle n'a pas d'effet avec une moyenne de 2 tubes par noeud comme pour la condition contrôle.

## Revendications

1. Aptamère comprenant un acide nucléique comprenant, ou constitué de :
- la séquence ACUGUCCCAGUAUGACGCGACUGCUUAGGUGGGAUGUUUCCC AUGCCUCG (SEQ ID NO : 1), ou
- une séquence comprenant, ou constituée d'au moins **25** nucléotides consécutifs d'une séquence ayant au moins 80% d'identité avec SEQ ID NO : 1, sous réserve qu'un acide nucléique constitué de cette séquence se lie à la protéine LAR, et le pourcentage d'identité entre deux séquences étant défini comme le nombre de positions pour lesquelles les bases sont identiques lorsque les séquences sont alignées de manière optimale, divisé par le nombre total de bases de la plus grande des deux séquences.

2. Aptamère selon la revendication 1, comprenant un acide nucléique constitué de la séquence GGGAGAUGCUCUGUCAGACUACGACUGUCCCAGUAUGACGCGACUGC UUAGGUGGGAUGUUUCCCAUGCCUCGACUGGCUGGCAGGUUGAUGACAAG (SEQ ID NO: 2).

3. Aptamère selon la revendication 1 ou 2, dans lequel l'acide nucléique est lié à au moins un groupement additionnel.

4. Aptamère selon la revendication 3, dans lequel le groupement additionnel est sélectionné dans le groupe constitué d'un marqueur détectable, d'un composé pharmacologique, et d'un composé susceptible de modifier les caractéristiques pharmacocinétiques d'un acide nucléique auquel il est lié.

5. Aptamère selon la revendication 3 ou 4, constitué de l'acide nucléique et d'au moins un groupement additionnel.

6. Aptamère selon l'une des revendications 1 à 5, pour son utilisation à titre de médicament ou d'agent diagnostique.

7. Composition pharmaceutique ou diagnostique comprenant, à titre de substance active, au moins un aptamère tel que défini dans l'une des revendications 1 à 5 en association avec au moins un véhicule pharmaceutiquement acceptable.

8. Utilisation *in vitro* d'un aptamère tel que défini dans l'une des revendications 1 à 5, pour détecter la présence de protéine LAR ou déterminer la quantité de protéine LAR dans un échantillon biologique.

9. Méthode *in vitro* de détection ou de quantification de protéine LAR dans un échantillon biologique, comprenant :
- mettre en contact l'échantillon biologique avec un aptamère tel que défini dans l'une des revendications 1 à 5 ;
- quantifier, ou détecter la présence ou l'absence d'aptamère fixé dans l'échantillon ;
- en déduire la quantité, ou la présence ou l'absence de protéine LAR dans l'échantillon.

10. Méthode selon la revendication 9, dans laquelle la détection d'aptamère fixé dans l'échantillon est effectuée par mise en oeuvre d'une RT-PCR destinée à amplifier l'aptamère.

11. Utilisation d'un aptamère tel que défini dans l'une des revendications 1 à 5, pour le criblage de ligands de protéine LAR.

12. Méthode de criblage de ligands de protéine LAR comprenant :
- la mise en présence de protéine LAR et, concomitamment ou successivement, d'un ligand à cribler et d'un aptamère tel que défini dans l'une des revendications 1 à 5 ;
- déterminer la quantité d'aptamère fixé à la protéine LAR ;
- en déduire si le ligand est un ligand de la protéine LAR.

## Patentansprüche

1. Aptamer, umfassend eine Nukleinsäure, welche umfasst oder gebildet ist aus:
- der Sequenz CUGUCCCAGUAUGACGCGACUGCUUAGGUGGGAUGUUU CCCAUGCCUCG (SEQ ID Nr. 1), oder
- einer Sequenz, welche umfasst oder gebildet ist aus wenigstens 25 aufeinanderfolgenden Nukleotiden einer Sequenz, welche zu mindestens 80% identisch ist mit SEQ ID Nr. 1, sofern sich eine aus dieser Sequenz gebildete Nukleinsäure an das LAR-Protein bindet und der Prozentsatz der Identität von zwei Sequenzen definiert ist als die Anzahl der Positionen, an denen die Basen identisch sind, wenn die Sequenzen optimal angeordnet sind, geteilt durch die Gesamtanzahl Basen der größeren der zwei Sequenzen.

2. Aptamer nach Anspruch 1, umfassend eine Nukleinsäure, welche gebildet ist aus der Sequenz

3. Aptamer nach Anspruch 1 oder 2, wobei die Nukleinsäure an wenigstens eine zusätzliche Gruppe gebunden ist.

4. Aptamer nach Anspruch 3, wobei die zusätzliche Gruppe gewählt ist aus der Gruppe, welche gebildet ist aus einem nachweisbaren Marker, einem pharmakologischen Bestandteil und einem Bestandteil, welcher die pharmakokinetischen Kennzeichen einer Nukleinsäure, an die er gebunden ist, verändern kann.

5. Aptamer nach Anspruch 3 oder 4, welches aus der Nukleinsäure und aus wenigstens einer zusätzlichen Gruppe gebildet ist.

6. Aptamer nach einem der Ansprüche 1 bis 5, für eine Verwendung als Medikament oder als diagnostisches Mittel.

7. Pharmazeutische oder diagnostische Zusammensetzung, welche als Wirkstoff wenigstens ein Aptamer, wie in einem der Ansprüche 1 bis 5 definiert, umfasst, in Verbindung mit wenigstens einem akzeptablen pharmazeutischen Träger.

8. In-vitro-Verwendung eines Aptamers, wie in einem der Ansprüche 1 bis 5 definiert, zur Erkennung des Vorliegens des LAR-Proteins oder zur Bestimmung der Menge des LAR-Proteins in einer biologischen Probe.

9. In-vitro-Verfahren zur Erkennung oder Quantifizierung des LAR-Proteins in einer biologischen Probe, umfassend:
- Inkontaktbringen der biologischen Probe mit einem Aptamer, wie in einem der Ansprüche 1 bis 5 definiert;
- quantitative Bestimmung oder Erkennung des Vorliegens oder der Abwesenheit des fixierten Aptamers in der Probe;
- Ableiten der Menge oder des Vorliegens oder der Abwesenheit des LAR-Proteins in der Probe.

10. Verfahren nach Anspruch 9, wobei die Erkennung des fixierten Aptamers in der Probe mittels Durchführung eines RT-PCR-Verfahrens erfolgt, mit dem Ziel, das Aptamer zu verstärken.

11. Verwendung eines Aptamers, wie in einem der Ansprüche 1 bis 5 definiert, für das Screening von LAR-Protein-Liganden.

12. Verfahren zum Screenen von LAR-Protein-Liganden, umfassend:
- Nachweis des LAR-Proteins und gleichzeitig oder aufeinanderfolgend eines zu screenenden Liganden oder eines Aptamers, wie in einem der Ansprüche 1 bis 5 definiert;
- Bestimmen der Menge des an dem LAR-Protein fixierten Aptamers;
- Ableiten, ob es sich bei dem Liganden um einen Liganden des LAR-Proteins handelt.

## Claims

1. Aptamer comprising a nucleic acid comprising or consisting of:
- the sequence ACUGUCCCAGUAUGACGCGACUGCUUAGGUGGGAUGUUUCCCAUGCCU CG (SEQ ID NO: 1), or
- a sequence comprising, or consisting of, at least 25 consecutive nucleotides of a sequence having at least 80% identity with SEQ ID NO: 1, subject to a nucleic acid consisting of this sequence binds to the LAR protein, and the percentage of identity between two sequences being defined as the number of positions for which the bases are identical when the sequences are optimally aligned, divided by the total number of bases of the larger of the two sequences.

2. Aptamer according to claim 1, comprising a nucleic acid consisting of the sequence

3. Aptamer according to claim 1 or 2, wherein the nucleic acid is bonded to at least one additional group.

4. Aptamer according to claim 3, wherein the additional group is selected from the group consisting of a detectable label, a pharmacological compound, and a compound capable of modifying the pharmacokinetic characteristics of a nucleic acid to which it is bonded.

5. Aptamer according to claim 3 or 4, consisting of the nucleic acid and at least one additional group.

6. Aptamer according to one of claims 1 to 5, for use as a medicament or a diagnostic agent.

7. Pharmaceutical or diagnostic composition comprising, as an active substance, at least one aptamer such as defined in one of the claims 1 to 5 in combination with at least one pharmaceutically acceptable vehicle.

8. *In vitro* use of an aptamer as defined in one of claims 1 to 5, for detecting the presence of LAR protein or determining the amount of LAR protein in a biological sample.

9. *In vitro* method of detecting or quantifying LAR protein in a biological sample, comprising:
- bringing the biological sample into contact with an aptamer as defined in one of claims 1 to 5;
- quantifying, or detecting the presence or absence of bound aptamer in the sample;
- deducing therefrom, the amount or the presence or absence of LAR protein in the sample.

10. Method according to claim 9, wherein the detection of bound aptamer in the sample is carried out by performing an RT-PCR intended to amplify the aptamer.

11. Use of an aptamer as defined in one of claims 1 to 5, for screening for LAR protein ligands.

12. Method of screening for LAR protein ligands comprising:
- bringing together LAR protein and, concomitantly or successively, a ligand to be screened and an aptamer such as defined in one of claims 1 to 5;
- determining the amount of aptamer bound to the LAR protein;
- deducing therefrom whether the ligand is a LAR protein ligand.


### LISTAGE DES SEQUENCES

<110> COMMISSARIAT A L'ENERGIE ATOMIQUE ET AUX ENERGIES
   ALTERNATIVE
<120> LIGAND SPECIFIQUE DE LA PROTEINE LAR
<130> F263-424
<160> 5
<170> PatentIn version 3.5
<210> 1
   <211> 50
   <212> RNA
   <213> Séquence artificielle
<220>
   <223> Aptamère anti-LAR
<400> 1
   acugucccag uaugacgcga cugcuuaggu gggauguuuc ccaugccucg 50
<210> 2
   <211> 97
   <212> RNA
   <213> Séquence artificielle
<220>
   <223> Aptamère anti-LAR
<400> 2
   gggagaugcu cugucagacu acgacugucc caguaugacg cgacugcuua ggugggaugu      60
   uucccaugcc ucgacuggcu ggcagguuga ugacaag                               97
<210> 3
   <211> 1907
   <212> PRT
   <213> Homo sapiens
<400> 3
   Met Ala Pro Glu Pro Ala Pro Gly Arg Thr Met Val Pro Leu Val Pro
   1               5                   10                  15
   Ala Leu Val Met Leu Gly Leu Val Ala Gly Ala His Gly Asp Ser Lys
               20                  25                  30
   Pro Val Phe Ile Lys Val Pro Glu Asp Gln Thr Gly Leu Ser Gly Gly
           35                  40                  45
   Val Ala Ser Phe Val Cys Gln Ala Thr Gly Glu Pro Lys Pro Arg Ile
       50                  55                  60
   Thr Trp Met Lys Lys Gly Lys Lys Val Ser Ser Gln Arg Phe Glu Val
   65                  70                  75                  80
   Ile Glu Phe Asp Asp Gly Ala Gly Ser Val Leu Arg Ile Gln Pro Leu
                   85                  90                  95
   Arg Val Gln Arg Asp Glu Ala Ile Tyr Glu Cys Thr Ala Thr Asn Ser
               100                 105                 110
   Leu Gly Glu Ile Asn Thr Ser Ala Lys Leu Ser Val Leu Glu Glu Glu
           115                 120                 125
   Gln Leu Pro Pro Gly Phe Pro Ser Ile Asp Met Gly Pro Gln Leu Lys
       130                 135                 140
   Val Val Glu Lys Ala Arg Thr Ala Thr Met Leu Cys Ala Ala Gly Gly
   145                 150                 155                 160
   Asn Pro Asp Pro Glu Ile Ser Trp Phe Lys Asp Phe Leu Pro Val Asp
                   165                 170                 175
   Pro Ala Thr Ser Asn Gly Arg Ile Lys Gln Leu Arg Ser Gly Ala Leu
               180                 185                 190
   Gln Ile Glu Ser Ser Glu Glu Ser Asp Gln Gly Lys Tyr Glu Cys Val
           195                 200                 205
   Ala Thr Asn Ser Ala Gly Thr Arg Tyr Ser Ala Pro Ala Asn Leu Tyr
       210                 215                 220
   Val Arg Val Arg Arg Val Ala Pro Arg Phe Ser Ile Pro Pro Ser Ser
   225                 230                 235                 240
   Gln Glu Val Met Pro Gly Gly Ser Val Asn Leu Thr Cys Val Ala Val
                   245                 250                 255
   Gly Ala Pro Met Pro Tyr Val Lys Trp Met Met Gly Ala Glu Glu Leu
               260                 265                 270
   Thr Lys Glu Asp Glu Met Pro Val Gly Arg Asn Val Leu Glu Leu Ser
           275                 280                 285
   Asn Val Val Arg Ser Ala Asn Tyr Thr Cys Val Ala Ile Ser Ser Leu
       290                 295                 300
   Gly Met Ile Glu Ala Thr Ala Gln Val Thr Val Lys Ala Leu Pro Lys
   305                 310                 315                 320
   Pro Pro Ile Asp Leu Val Val Thr Glu Thr Thr Ala Thr Ser Val Thr
                   325                 330                 335
   Leu Thr Trp Asp Ser Gly Asn Ser Glu Pro Val Thr Tyr Tyr Gly Ile
               340                 345                 350
   Gln Tyr Arg Ala Ala Gly Thr Glu Gly Pro Phe Gln Glu Val Asp Gly
           355                 360                 365
   Val Ala Thr Thr Arg Tyr Ser Ile Gly Gly Leu Ser Pro Phe Ser Glu
       370                 375                 380
   Tyr Ala Phe Arg Val Leu Ala Val Asn Ser Ile Gly Arg Gly Pro Pro
   385                 390                 395                 400
   Ser Glu Ala Val Arg Ala Arg Thr Gly Glu Gln Ala Pro Ser Ser Pro
                   405                 410                 415
   Pro Arg Arg Val Gln Ala Arg Met Leu Ser Ala Ser Thr Met Leu Val
               420                 425                 430
   Gln Trp Glu Pro Pro Glu Glu Pro Asn Gly Leu Val Arg Gly Tyr Arg
           435                 440                 445
   Val Tyr Tyr Thr Pro Asp Ser Arg Arg Pro Pro Asn Ala Trp His Lys
       450                 455                 460
   His Asn Thr Asp Ala Gly Leu Leu Thr Thr Val Gly Ser Leu Leu Pro
   465                 470                 475                 480
   Gly Ile Thr Tyr Ser Leu Arg Val Leu Ala Phe Thr Ala Val Gly Asp
                   485                 490                 495
   Gly Pro Pro Ser Pro Thr Ile Gln Val Lys Thr Gln Gln Gly Val Pro
               500                 505                 510
   Ala Gln Pro Ala Asp Phe Gln Ala Glu Val Glu Ser Asp Thr Arg Ile
           515                 520                 525
   Gln Leu Ser Trp Leu Leu Pro Pro Gln Glu Arg Ile Ile Met Tyr Glu
       530                 535                 540
   Leu Val Tyr Trp Ala Ala Glu Asp Glu Asp Gln Gln His Lys Val Thr
   545                 550                 555                 560
   Phe Asp Pro Thr Ser Ser Tyr Thr Leu Glu Asp Leu Lys Pro Asp Thr
                   565                 570                 575
   Leu Tyr Arg Phe Gln Leu Ala Ala Arg Ser Asp Met Gly Val Gly Val
               580                 585                 590
   Phe Thr Pro Thr Ile Glu Ala Arg Thr Ala Gln Ser Thr Pro Ser Ala
           595                 600                 605
   Pro Pro Gln Lys Val Met Cys Val Ser Met Gly Ser Thr Thr Val Arg
       610                 615                 620
   Val Ser Trp Val Pro Pro Pro Ala Asp Ser Arg Asn Gly Val Ile Thr
   625                 630                 635                 640
   Gln Tyr Ser Val Ala Tyr Glu Ala Val Asp Gly Glu Asp Arg Gly Arg
                   645                 650                 655
   His Val Val Asp Gly Ile Ser Arg Glu His Ser Ser Trp Asp Leu Val
               660                 665                 670
   Gly Leu Glu Lys Trp Thr Glu Tyr Arg Val Trp Val Arg Ala His Thr
           675                 680                 685
   Asp Val Gly Pro Gly Pro Glu Ser Ser Pro Val Leu Val Arg Thr Asp
       690                 695                 700
   Glu Asp Val Pro Ser Gly Pro Pro Arg Lys Val Glu Val Glu Pro Leu
   705                 710                 715                 720
   Asn Ser Thr Ala Val His Val Tyr Trp Lys Leu Pro Val Pro Ser Lys
                   725                 730                 735
   Gln His Gly Gln Ile Arg Gly Tyr Gln Val Thr Tyr Val Arg Leu Glu
               740                 745                 750
   Asn Gly Glu Pro Arg Gly Leu Pro Ile Ile Gln Asp Val Met Leu Ala
           755                 760                 765
   Glu Ala Gln Trp Arg Pro Glu Glu Ser Glu Asp Tyr Glu Thr Thr Ile
       770                 775                 780
   Ser Gly Leu Thr Pro Glu Thr Thr Tyr Ser Val Thr Val Ala Ala Tyr
   785                 790                 795                 800
   Thr Thr Lys Gly Asp Gly Ala Arg Ser Lys Pro Lys Ile Val Thr Thr
                   805                 810                 815
   Thr Gly Ala Val Pro Gly Arg Pro Thr Met Met Ile Ser Thr Thr Ala
               820                 825                 830
   Met Asn Thr Ala Leu Leu Gln Trp His Pro Pro Lys Glu Leu Pro Gly
           835                 840                 845
   Glu Leu Leu Gly Tyr Arg Leu Gln Tyr Cys Arg Ala Asp Glu Ala Arg
       850                 855                 860
   Pro Asn Thr Ile Asp Phe Gly Lys Asp Asp Gln His Phe Thr Val Thr
   865                 870                 875                 880
   Gly Leu His Lys Gly Thr Thr Tyr Ile Phe Arg Leu Ala Ala Lys Asn
                   885                 890                 895
   Arg Ala Gly Leu Gly Glu Glu Phe Glu Lys Glu Ile Arg Thr Pro Glu
               900                 905                 910
   Asp Leu Pro Ser Gly Phe Pro Gln Asn Leu His Val Thr Gly Leu Thr
           915                 920                 925
   Thr Ser Thr Thr Glu Leu Ala Trp Asp Pro Pro Val Leu Ala Glu Arg
       930                 935                 940
   Asn Gly Arg Ile Ile Ser Tyr Thr Val Val Phe Arg Asp Ile Asn Ser
   945                 950                 955                 960
   Gln Gln Glu Leu Gln Asn Ile Thr Thr Asp Thr Arg Phe Thr Leu Thr
                   965                 970                 975
   Gly Leu Lys Pro Asp Thr Thr Tyr Asp Ile Lys Val Arg Ala Trp Thr
               980                 985                 990
   Ser Lys Gly Ser Gly Pro Leu Ser  Pro Ser Ile Gln Ser  Arg Thr Met
           995                 1000                1005
   Pro Val  Glu Gln Val Phe Ala  Lys Asn Phe Arg Val  Ala Ala Ala
       1010                 1015                 1020
   Met Lys  Thr Ser Val Leu Leu  Ser Trp Glu Val Pro  Asp Ser Tyr
       1025                 1030                 1035
   Lys Ser  Ala Val Pro Phe Lys  Ile Leu Tyr Asn Gly  Gln Ser Val
       1040                 1045                 1050
   Glu Val  Asp Gly His Ser Met  Arg Lys Leu Ile Ala  Asp Leu Gln
       1055                 1060                 1065
   Pro Asn  Thr Glu Tyr Ser Phe  Val Leu Met Asn Arg  Gly Ser Ser
   1070 1075 1080
   Ala Gly Gly Leu Gln His Leu Val Ser Ile Arg Thr Ala Pro Asp
   1085 1090 1095
   Leu Leu Pro His Lys Pro Leu Pro Ala Ser Ala Tyr Ile Glu Asp
   1100 1105 1110
   Gly Arg Phe Asp Leu Ser Met Pro His Val Gln Asp Pro Ser Leu
   1115 1120 1125
   Val Arg Trp Phe Tyr Ile Val Val Val Pro Ile Asp Arg Val Gly
   1130 1135 1140
   Gly Ser Met Leu Thr Pro Arg Trp Ser Thr Pro Glu Glu Leu Glu
   1145 1150 1155
   Leu Asp Glu Leu Leu Glu Ala Ile Glu Gln Gly Gly Glu Glu Gln
   1160 1165 1170
   Arg Arg Arg Arg Arg Gln Ala Glu Arg Leu Lys Pro Tyr Val Ala
   1175 1180 1185
   Ala Gln Leu Asp Val Leu Pro Glu Thr Phe Thr Leu Gly Asp Lys
   1190 1195 1200
   Lys Asn Tyr Arg Gly Phe Tyr Asn Arg Pro Leu Ser Pro Asp Leu
   1205 1210 1215
   Ser Tyr Gln Cys Phe Val Leu Ala Ser Leu Lys Glu Pro Met Asp
   1220 1225 1230
   Gln Lys Arg Tyr Ala Ser Ser Pro Tyr Ser Asp Glu Ile Val Val
   1235 1240 1245
   Gln Val Thr Pro Ala Gln Gln Gln Glu Glu Pro Glu Met Leu Trp
   1250 1255 1260
   Val Thr Gly Pro Val Leu Ala Val Ile Leu Ile Ile Leu Ile Val
   1265 1270 1275
   Ile Ala Ile Leu Leu Phe Lys Arg Lys Arg Thr His Ser Pro Ser
   1280 1285 1290
   Ser Lys Asp Glu Gln Ser Ile Gly Leu Lys Asp Ser Leu Leu Ala
   1295 1300 1305
   His Ser  Ser Asp Pro Val Glu  Met Arg Arg Leu Asn  Tyr Gln Thr
       1310                 1315                 1320
   Pro Gly  Met Arg Asp His Pro  Pro Ile Pro Ile Thr  Asp Leu Ala
       1325                 1330                 1335
   Asp Asn  Ile Glu Arg Leu Lys  Ala Asn Asp Gly Leu  Lys Phe Ser
       1340                 1345                 1350
   Gln Glu  Tyr Glu Ser Ile Asp  Pro Gly Gln Gln Phe  Thr Trp Glu
       1355                 1360                 1365
   Asn Ser  Asn Leu Glu Val Asn  Lys Pro Lys Asn Arg  Tyr Ala Asn
       1370                 1375                 1380
   Val Ile  Ala Tyr Asp His Ser  Arg Val Ile Leu Thr  Ser Ile Asp
       1385                 1390                 1395
   Gly Val  Pro Gly Ser Asp Tyr  Ile Asn Ala Asn Tyr  Ile Asp Gly
       1400                 1405                 1410
   Tyr Arg  Lys Gln Asn Ala Tyr  Ile Ala Thr Gln Gly  Pro Leu Pro
       1415                 1420                 1425
   Glu Thr  Met Gly Asp Phe Trp  Arg Met Val Trp Glu  Gln Arg Thr
       1430                 1435                 1440
   Ala Thr  Val Val Met Met Thr  Arg Leu Glu Glu Lys  Ser Arg Val
       1445                 1450                 1455
   Lys Cys  Asp Gln Tyr Trp Pro  Ala Arg Gly Thr Glu  Thr Cys Gly
       1460                 1465                 1470
   Leu Ile  Gln Val Thr Leu Leu  Asp Thr Val Glu Leu  Ala Thr Tyr
       1475                 1480                 1485
   Thr Val  Arg Thr Phe Ala Leu  His Lys Ser Gly Ser  Ser Glu Lys
       1490                 1495                 1500
   Arg Glu  Leu Arg Gln Phe Gln  Phe Met Ala Trp Pro  Asp His Gly
       1505                 1510                 1515
   Val Pro  Glu Tyr Pro Thr Pro  Ile Leu Ala Phe Leu  Arg Arg Val
       1520                 1525                 1530
   Lys Ala  Cys Asn Pro Leu Asp  Ala Gly Pro Met Val  Val His Cys
       1535                 1540                 1545
   Ser Ala  Gly Val Gly Arg Thr  Gly Cys Phe Ile Val  Ile Asp Ala
       1550                 1555                 1560
   Met Leu  Glu Arg Met Lys His  Glu Lys Thr Val Asp  Ile Tyr Gly
       1565                 1570                 1575
   His Val  Thr Cys Met Arg Ser  Gln Arg Asn Tyr Met  Val Gln Thr
       1580                 1585                 1590
   Glu Asp  Gln Tyr Val Phe Ile  His Glu Ala Leu Leu  Glu Ala Ala
       1595                 1600                 1605
   Thr Cys  Gly His Thr Glu Val  Pro Ala Arg Asn Leu  Tyr Ala His
       1610                 1615                 1620
   Ile Gln  Lys Leu Gly Gln Val  Pro Pro Gly Glu Ser  Val Thr Ala
       1625                 1630                 1635
   Met Glu  Leu Glu Phe Lys Leu  Leu Ala Ser Ser Lys  Ala His Thr
       1640                 1645                 1650
   Ser Arg  Phe Ile Ser Ala Asn  Leu Pro Cys Asn Lys  Phe Lys Asn
       1655                 1660                 1665
   Arg Leu  Val Asn Ile Met Pro  Tyr Glu Leu Thr Arg  Val Cys Leu
       1670                 1675                 1680
   Gln Pro  Ile Arg Gly Val Glu  Gly Ser Asp Tyr Ile  Asn Ala Ser
       1685                 1690                 1695
   Phe Leu  Asp Gly Tyr Arg Gln  Gln Lys Ala Tyr Ile  Ala Thr Gln
       1700                 1705                 1710
   Gly Pro  Leu Ala Glu Ser Thr  Glu Asp Phe Trp Arg  Met Leu Trp
       1715                 1720                 1725
   Glu His  Asn Ser Thr Ile Ile  Val Met Leu Thr Lys  Leu Arg Glu
       1730                 1735                 1740
   Met Gly  Arg Glu Lys Cys His  Gln Tyr Trp Pro Ala  Glu Arg Ser
       1745                 1750                 1755
   Ala Arg  Tyr Gln Tyr Phe Val  Val Asp Pro Met Ala  Glu Tyr Asn
       1760                 1765                 1770
   Met Pro  Gln Tyr Ile Leu Arg  Glu Phe Lys Val Thr  Asp Ala Arg
       1775                 1780                 1785
   Asp Gly  Gln Ser Arg Thr Ile  Arg Gln Phe Gln Phe  Thr Asp Trp
       1790                 1795                 1800
   Pro Glu  Gln Gly Val Pro Lys  Thr Gly Glu Gly Phe  Ile Asp Phe
       1805                 1810                 1815
   Ile Gly  Gln Val His Lys Thr  Lys Glu Gln Phe Gly  Gln Asp Gly
       1820                 1825                 1830
   Pro Ile  Thr Val His Cys Ser  Ala Gly Val Gly Arg  Thr Gly Val
       1835                 1840                 1845
   Phe Ile  Thr Leu Ser Ile Val  Leu Glu Arg Met Arg  Tyr Glu Gly
       1850                 1855                 1860
   Val Val  Asp Met Phe Gln Thr  Val Lys Thr Leu Arg  Thr Gln Arg
       1865                 1870                 1875
   Pro Ala  Met Val Gln Thr Glu  Asp Gln Tyr Gln Leu  Cys Tyr Arg
       1880                 1885                 1890
   Ala Ala  Leu Glu Tyr Leu Gly  Ser Phe Asp His Tyr  Ala Thr
       1895                 1900                 1905
<210> 4
   <211> 24
   <212> DNA
   <213> Séquence artificielle
<220>
   <223> Amorce PCR
<400> 4
   cttgtcatca acctgccagc cagt 24
<210> 5
   <211> 23
   <212> DNA
   <213> Séquence artificielle
<220>
   <223> Amorce PCR
<400> 5
   gggagatgct ctgtcagact acg 23